(19) Europäisches Patentamt
European Patent Office
Office européen des brevets

(11) EP 0 785 952 B1

(12) **FASCICULE DE BREVET EUROPEEN**

(45) Date de publication et mention de la délivrance du brevet:
**15.07.1998 Bulletin 1998/29**

(21) Numéro de dépôt: **95935487.9**

(22) Date de dépôt: **13.10.1995**

(51) Int Cl.[6]: **C08B 37/08**, A61K 7/48

(86) Numéro de dépôt international:
**PCT/FR95/01346**

(87) Numéro de publication internationale:
**WO 96/11956 (25.04.1996 Gazette 1996/18)**

# (54) CERAMIDE GREFFEE A PROPRIETE ANTI-ELASTASE

GEPFROPFTES CERAMID MIT ANTIELASTASEWIRKUNG

GRAFT CERAMIDE HAVING AN ANTI-ELASTASE PROPERTY

(84) Etats contractants désignés:
**AT BE CH DE DK ES FR GB GR IE IT LI LU MC NL PT SE**

(30) Priorité: **14.10.1994 FR 9412289**

(43) Date de publication de la demande:
**30.07.1997 Bulletin 1997/31**

(73) Titulaire: **PIERRE FABRE DERMO-COSMETIQUE**
**92100 Boulogne (FR)**

(72) Inventeurs:
- **LACOSTE, Lydie**
  **F-31120 Roquettes (FR)**
- **MSIKA, Philippe**
  **F-75018 PARIS (FR)**
- **NAVARRO, Roger**
  **F-09100 Pamiers (FR)**
- **CHARVERON, Marie**
  **31000 Toulouse (FR)**
- **PERRIER, Eric**
  **F-38138 LES COTES D'AREY (FR)**

(74) Mandataire: **Ahner, Francis**
**CABINET REGIMBEAU**
**26, avenue Kléber**
**75116 Paris (FR)**

(56) Documents cités:
**EP-A- 0 493 622**

- **PATENT ABSTRACTS OF JAPAN vol. 13 no. 205 (C-595) [3553] ,15 Mai 1989 & JP,A,01 022812 (KANEBO LTD) 25 Janvier 1989,**
- **PATENT ABSTRACTS OF JAPAN vol. 13 no. 205 (C-595) [3553] ,15 Mai 1989 & JP,A,01 022809 (KANEBO) 25 Janvier 1989,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 785 952 B1

## Description

La présente invention se rapporte à de nouveaux conjugués à propriété anti-élastase, ces molécules étant obtenues en greffant une chondroïtine sulfate et un céramide.

L'utilisation de ce type d'activité anti-élastase en dermo-cosmétologie revêt un grand intérêt pour combattre le vieillissement cutané, notamment photo-induit.

### I. RAPPEL SUR L'ELASTINE

L'élastine est une protéine fibreuse. Associée aux glycoprotéines de structure, elle forme la fibre élastique, responsable de l'élasticité des tissus. On différencie, en fait, trois types de réseaux élastiques :

- un réseau de fibres oxytalanes qui s'ancrent dans la lame basale dermoépidermique
- ces fines fibrilles se prolongent par un réseau de fibres élaunines qui sont parallèles à la jonction dermo-épidermique
- plus en profondeur, elles s'anastomosent pour donner les fibres orcéinophiles, qui sont considérées comme les fibres élastiques matures. Elles sont orientées perpendiculairement à la lame basale et "plongent" dans le derme profond.

Au cours du vieillissement, on observe une raréfaction de ces fibres, la première manifestation étant la disparition des fibres oxytalanes. On observe donc un "effondrement" de la jonction dermo-épidermique, la peau devient flasque. Il faut, en effet, avoir présent à l'esprit que l'organisme synthétise l'élastine dès la vie embryonnaire et durant toute la croissance jusqu'à l'âge de 20 ans environ. Mais, dès l'âge adulte, la synthèse d'élastine devient faible et très lente. A ce phénomène vient s'ajouter une destruction de l'élastine par des élastases, enzymes protéolytiques dont l'activité augmente avec l'age. Par ailleurs, l'exposition chronique au soleil provoque des altérations qui viennent s'ajouter au vieillissement physiologique. Les fibres élastiques, sous l'effet des UV, deviennent plus épaisses et plus courtes, elles se transforment en une masse amorphe : on parle l'élastose actinique.

Tous ces mécanismes sont donc en faveur de produits qui protègeraient le capital élastique de la peau, notamment grâce à des bloqueurs de l'activité élastase, la biosynthèse de l'élastine étant, quant à elle, très lente.

### II. RAPPEL SUR L'ELASTASE

Pour pouvoir bloquer les élastases, il faut connaître leur mode de fonctionnement. Les élastases relèvent apparemment de plusieurs catégories d'enzymes. La plus importante paraît être l'élastase produite par les fibroblastes cutanés eux-mêmes. Il s'agit d'une métalloprotéase à zinc, à localisation membranaire. Une autre source potentielle d'élastases est représentée par les leucocytes véhiculés par le sang. Les leucocytes polynucléaires portent dans leurs granules une sérine-protéase puissante (l'élastase leucocytaire) pouvant attaquer non seulement les fibres élastiques mais pratiquement tous les constituants de la matrice extra-cellulaire.

Cette enzyme est particulièrement activée dans les phénomènes inflammatoires, qui peuvent notamment etre observés après une exposition aux U.V.

Les élastases possèdent dans leur structure un site de fixation et un site actif. L'élastine vient se fixer sur les deux sites, l'un assurant le contact, l'autre la coupure de l'élastine. Les inhibiteurs de l'élastase se fixent soit sur le site actif, soit sur le site de fixation.

La demanderesse a, grace à un greffage spécifique de glycosaminoglycanes (GAG), en particulier de chondroitine sulfate et de céramide, cherché à agir sur les deux sites, comme cela va etre maintenant expliqué.

### III - RAPPELS SUR LES CERAMIDES

La partie lipidique de l'épiderme est particulièrement riche en céramides, en cholestérol et en acides gras libres.

Les céramides occupent une place majeure dans les couches supérieures de cet épiderme. Ils constituent une classe particulière de sphingolipides qui comprennent une ou plusieurs fonctions hydroxy, amide et/ou ester, les fonctions hydroxy pouvant être éventuellement estérifiées. Leur formule générale peut se représenter comme suit :

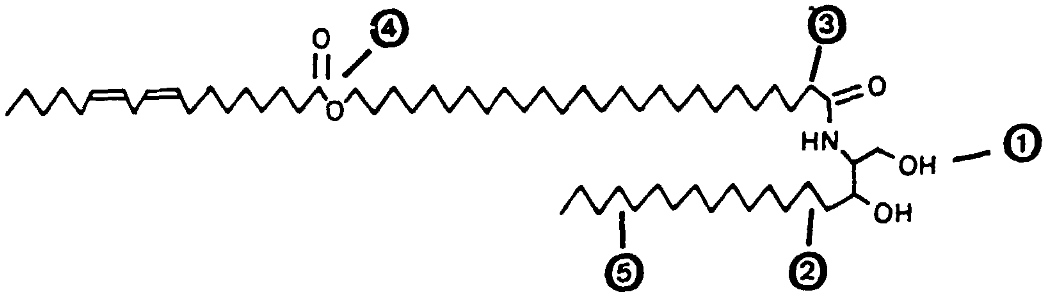

Les points clés de cette formule mettent en évidence :

- en position 1 : les céramides ne sont pas glycosylés comme les sphingolipides.
- en position 2 : il peut y avoir une double liaison ou un groupe -OH.
- en position 3 : on retrouve parfois un groupe -OH
- en position 4: les ω-hydroxyacides sont souvent estérifiés : acylcéramides.
- en position 5 : la longueur de chaîne est variable.

Le role des céramides dans le ciment intercornéocytaire a été largement étudié ces demières années. Il est apparu une triple fonction des céramides :

- dans la régulation du flux de perte d'eau transépidermique ;
- dans la cohésion des couches supérieures de l'épiderme ; et
- dans la résistance aux agressions extérieures.

Une 4ème fonction a été décrite pour des céramides d'origine végétale: il s'agit d'une activité anti-élastase qui peut être mise en évidence selon deux modèles in vitro :

- inhibition de l'élastase leucocytaire vis à vis d'un substrat synthétique (MeOSuc $(Ala)_2$ Pro Valparanitroanilide) ; et
- inhibition de l'élastase leucocytaire vis à vis d'un substrat naturel (élastine radiomarquée).

Les inhibitions 50% (1C50) sont identiques dans les deux cas et voisines de 30 µg/ml.

Par analogie, la Demanderesse a pour sa part étudié l'activité anti-élastase de céramides de synthèse, notamment répondant à la structure chimique suivante :

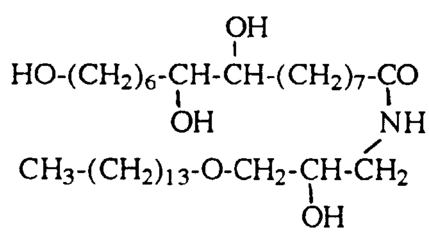

Cette molécule présente l'avantage d'être chimiquement définie, alors que les céramides végétaux sont en fait des mélanges structuraux.

Son comportement théorique au niveau moléculaire met en évidence un parfait alignement de ses deux chaînes grasses, traduisant une conformation équivalente à celles des céramides naturels. Cette structure en position de "rivet" permet d'expliquer l'intégration de ce céramide de synthèse au niveau du ciment intercornéocytaire. Ce produit est commercialisé par la société SEDERMA sous le nom de CERAMIDES H03.

L'activité anti-élastase de ce céramide est objectivée par une technique in vitro, basée sur la diffusion d'élastase dans un gel d'agarose contenant de l'élastine insoluble colorée par l'orcéine et dispersée dans le gel. Cette méthode sera détaillée ulterieurement (paragraphe VI).

Selon cette technique, le céramide de synthèse testé à 0,5% inhibe totalement l'activité de l'élastase (100% d'inhibition) alors que les céramides vegétaux précédemment cités, testés à la meme dose 0,5%, n'inhibent qu'à 60% l'activité de l'élastase.

La Demanderesse en a conclu à l'intérêt des céramides, ou de mélange dérivés de céramides, de toute origine :

- animale : exemple : SPHINGOLIPID CB1, commercialisés par la société NIKKO CHEMICALS,
- végétale: exemple: CERAMIDES VEGETALES de INOCOSM,
- issue de biotechnologie : exemple : CERAMIDE 3 DERIVATIVES de GIST-BROCADES,
- chimique : exemple : CERAMIDES H03 de SEDERMA cene liste étant non exhaustive...

Intéret dans la protection du capital élastique de la peau, en complément à leur action sur la cohésion intercornéocytaire.

Toutefois, cette action antii-élastase est probablement due à une protection des fibres élastiques par les céramides, étant donné que les fibres élastiques ont une forte affinité pour les composés lipophiles; les céramides agissant plus alors par protection des fibres élastiques que par une action anti-élastase directe. Ils empêchent l'attaque enzymatique par un effet barrière, de protection des fibres élastiques.

Mais, dans la présente invention, les céramides ne sont pas seuls responsables de l'activité anti-elastase, etant donné qu'ils sont couplés avec des glycosaminoglycanes, notamment avec une chondroîtine sulfate.

## IV GLYCOSAMINOGLYCANES et ACTIVITE ANTI-ELASTASE

Les glycosamlnoglycanes (G.A.G. en abrégé) sont des composants importants des systèmes de soutien extracellulaire des tissus. Ils jouent un role primordial dans l'hydratation et le maintien de la structure dermique.

Les G.A.G. sont formés d'unités disaccharidiques répétitives composées par l'association d'une N- acétyl hexosamine et d'un acide uronique.

Dans le derme, 100 à 150 chaînes de G.A.G. sont liées par liaison covalente à une protéine "porteuse". Cet ensemble constitue le protéoglycane. Dans certains cas, jusqu'à 100 protéoglycanes peuvent être liés de façon non covalente à une chaîne d'acide hyaluronique par le biais de glycoprotéines de liaison. Les chaînes de G.A.G. adoptent alors une forme étirée par répulsion électrostatique, et tendent à s'écarter les unes des autres, occupant ainsi un espace considérable.

Au cours du vieillissement, on observe une diminution de la teneur globale en G.A.G., ceci étant lié avec une perte d'hydratation et une diminution de l'épaisseur du derme.

Dans la grande variété G.A.G., les héparines sont connues pour leur activité inhibitrice de l'élastase lecocytaire (2). Nous nous sommes intéressés, en ce qui nous concerne à une autre famille de G.A.G., les chondroïtines sulfates, dont l'activité anti-élastase est également reconnue, sont particulièrement intéressantes.

Nous avons particulièrement étudié la chondroitine 4 - sulfate, commercialisée sous forme de sel de sodium par la société UNIPEX et extraite de cartilage de requin, des extraits de cartilage de requin ayant éte décrits comme ayant des propriétés inhibitrices d'enzyme (3).

Nous avons recherché l'activité anti-élastase de la chondroïtine 4 - sulfate selon la methode précédemment utilisée pour objectiver l'activité des céramides.

Nous avons obtenu avec 1% de chondroîtine 4 - sulfate une inhibition de 18,2% de l'activité de l'élastase.

Cette activité anti-élastase compétitive des chondroïtines sulfates, et des G.A.G. d'une manière plus générale, vient renforccr leur intérêt dans la protection du derme, en complément à leur rôle d'agent de soutien et de régulateur des échanges métaboliques au sein de la structure dermique. Il faut notamment rappeler la diminution de la teneur globale en G.A.G. observée au cours du vieillissement.

## V GREFFAGE

Formule générale des produits avant réaction :

| Glycosaminoglycannes : | Céramides : | Chlorure d'acide : |
|---|---|---|
| $R1(OH)_x(NH2)_y$ | $R_2(OH)_z$ | $R3\ (COCl)_w$ |
| | | Isocyanate : |
| $R_1$ est un polyose | | $R_4(NCO)_w$ |
| x et y sont supérieurs ou égaux à 1 | z est supérieur ou égal à 1 | w est supérieur ou égal à 2 |

Formule générale des produits après réaction :

R5(U-CO-R6-CO-O-R2)n

dans laquelle

n est supérieur ou égal à 1
R2-O représente un résidu céramide,
R5 représente un résidu GAG, et
R6 représente un agent pontant.

Lorsque les fonctions alcools primaires du GAG sont impliquées dans la réaction, alors (-U-) représente (-CH2-O-).
Lorsque les fonctions alcools secondaires du GAG sont impliquées dans la réaction, alors (-U-) représente (-O-).
Lorsque les fonctions amines du GAG sont impliquées dans la réaction, alors (-U-) représente (-NH-).

Type de liaisons identifiables dans les produits de la réaction

Outre les liaisons covalentes caractéristiques du produit de la réaction (liaisons esters, liaisons amides), des liaisons ioniques (entre les fonctions amines du GAG et les fonctions acides et alcools du céramide et de l'acide sébacique) et des liaisons hydrophobes sont également présentes dans le mélange réactionnel. Ces liaisons hydrophobes sont particulièrement fortes après élimination de l'eau utilisée dans le milieu réactionnel, et plus particulièrement lorsque le séchage est réalisé par lyophilisation, cette étape permettant l'élimination de l'eau libre et de l'eau liée asociée au produit.

Aspects inventifs du procédé

Les réactions chimiques qui sont réalisées à l'aide de poly-chlorures d'acides ou de poly-isocyanates, sont habituellement réalisées dans des milieux solvants susceptibles de piéger les protons libérés au cours de la réaction (pyridine, TEA, aniline, etc.), à des températures élevées.

Dans de tels solvants, les glycosaminoglycannes qui sont des macromolécules très hydrophiles ne se solubilisent pas et s'ils se solubilisaient, seraient partiellement dégradée par des températures élevées.

Or, les poly-chlorures d'acides ou les poly-isocyanates ne sont jamais utilisés dans les solvants aqueux car il réagissent avec l'eau pour donner des formes chimiques non réactives.

Les inventeurs ont néanmoins utilisés ces réactifs pour réaliser des réactions de greffage en phase aqueuse. Ils ont observé que ces réactifs ne réagissaient pas de facçon très rapide avec l'eau et que les ractions de greffages étaient prépondérantes lorsque des macromolécules étaient placées à réagir dans le milieu réactionnel.

L'avantage de cette technique de greffage devient alors évidente : aucun résidu de solvant, aucune dénaturation thermique des différents réactifs utilisés, innocuité totale des produits de réaction...

De plus, les sous-produits de la réaction (sébaçate disodique, chlorure de sodium) sont acceptables pour une utilisation cosmétique, sont parfaitement tolérés par la peau et les muqueuses, et ne sont pas éliminés des produits de la réaction, ce qui évite des étapes de purification complexe et coûteuses.

Les propriétés physico-chimiques mises en évidence lors de l'utilisation cosmétique des produits issus de la réaction sont directement liées à l'hydrophobicité apportée par la modification des glycosaminoglycannes réalisée lors de la réaction de greffage :

- solubilisés à chaud en phase aqueuse, les produits de la réaction sont émulsionnants ; ils créent des strucutres maillées amphiphiles de très grande taille dans la phase hydrophile des émulsions, qui s'intercalent et stabilisent les gouttelettes de la phase huileuse.
- ils peuvent être solubilisés à chaud en phase huileuse, ce qui n'est pas réalisable avec les glycosaminoglycannes, et donner des phases huileuses épaissies voire gélifiées lorsqu'ils sont utilisés à des concentrations élevées. Cette propnété est particulièrement intéressante puisque les produits de la réaction sont des hydratants puissants, que chacun de leurs constituants est habituellement utilisé dans des émulsions, et que grâce à leur couplage, ils peuvent être utilisés dans des formulations anhydres (rouges à lèvres, produits de maquillage ...) où il est habituellement particulièrement difficiel d'incorporer des macromolécules hydrophiles.

La présente invention concerne donc également un conjugué de céramides et de glycosaminoglycanes (GAG) obtenu par la réaction de couplage d'une céramide et d'une glycosaminoglycane (GAG) en présence d'un agent de

couplage de type diacide carboxylique, de formule HOOC-R6-COOH dans laquelle R6 représente un reste hydrocarboné en $C_1$-$C_{20}$, le conjugué obtenu comprenant des liaisons céramide/GAG de type covalentes (esters ou amides) et/ou de type ioniques (entre les fonctions amino du GAG et les fonctions acide du céramide) et/ou de type hydrogène, et/ou de type hydrophobe.

Elle concerne également une compositon cosmétique comprenant le conjugué selon l'invention et un support cosmétiquement acceptable.

**EXEMPLES DE FABRICATION D'UN PRINCIPE ACTIF BIFONCTIONNEL : CÉRAMIDE H03 GREFFÉ SUR CHONDROÏTINE 6 SULFATE MARINE**

Parmi les sphingolipides utilisés en cosmétologie, le Céramide H03 (Sederma) est particulièrement intéressant dans le cadre de l'invention puisqu'il possède qu'il possède une fonction alcool primaire, susceptible d'être utilisée au cours de la réaction de greffage.

**EXEMPLE 1 : Greffage par l'intermédiaire du dichlorure d'acide sébacique**

- Phase A : 570 g de Céramide H03 (soit environ 1 mole) sont placés dans 250g de dichlorure d'acide sébacique (soit environ 1 mole). L'ensemble est malavé pendant quelques minutes à température ambiante, puis porté à 80°C sous agitation pendant 10 minutes sous hotte.
- Phase B : 500 g de chondroitine sulfate marine - dont 98% en poids a une masse moléculaire supérieure à 10.000 D - sont dipsersés sous agitation modérée dans 10 litres d'eau déminéralisée. Le pH de la solution est alors ajustée à 10 à l'aide de soude 6N.

Après 30 minutes, lorsque la solution est totalement homogène, la phase A est versée très lentement, sous très forte agitation - du type ultraturrax, 20.000 rpm - dans la phase B. Tout au long de la réaction, réalisée à température ambiante, le pH est ajusté etnre 8 et 10 par ajout de soude 6N.

Après une agitation de ce type pendant 30 minutes, le mélange réactionnel est conservé pendant 2 heures sous agitation modérée, puis neutralisé au pH désiré (par exemple à pH 6,5) par HCl, 6N.

L'ensemble est alors dialysé à l'aide de membranes possédant un seuil de coupure de 5.000 D. Une fois les sels - du type chlorure de sodium - éliminés, le produit est alors lyophilisé puis stérilisé par rayonnement gamma. Il se présente alors sous la forme d'une fine poudre pulvérulente, non collante et non grasse, et peut ensuite être utilisée dans toutes les formulations cosmétiques puisqu'il peut être incorporé à la fois dans les phases aqueuses et dans les phases huileuses.

**EXEMPLE 1a**

Même chose que ci-dessus mais dans ce cas, des proportions différentes en chacun des trois constituants sont utilisées : en particulier, il peut être intéressant d'utiliser un excès de dichlorure d'acide sébacique de façon :

- 1) à utiliser ce réactif comme solubilisant des Céramides H03,
- 2) à améliorer les rendements du greffage.

Exemples :

- 100 g de céramides H03, 300 g de dichlorure d'acide sébacique, 100 g de chondroïtines sulfates marines dans 3 litres d'eau,

ou

- 100 g de céramides H03, 700 g de dichlorure d'acide sébacique, 100 g de chondroïtines sulfates marines dans 5 litres d'eau.

**EXEMPLE 1b**

Même chose que dans les exemples ci-dessus mais dans ce cas, toute l'opération est effectuée à 80-90°C. Les rendements de greffage sont améliorés. Le produit réalisé se présente sous la forme d'une pâte hétérogène, plus difficile à manipuler dans les formulations cosmétiques.

## VI <u>METHODE D'OBJECTIVATION</u>

La méthode d'objectivation déjà abordée dans les chapitre précédents va être maintenant décrite: elle est basée sur la diffusion d'elastase pancreatique dans un gel d'agarose contenant de l'élastine insoluble colorée par l'orcéïne et dispersée dans le gel.

Il s'agit d'une adaptation de la méthode "Visualization of proelastase in polyacrylamide gels", Analytical Biochemistry 83, 315 - 318, 1977 - Jan DIJKHOF - CEES POORT.

Les réactifs utilisés sont les suivants :

- Agar BIOMERIEUX réf.: 5.3041
- TRIS, (hydroxyméthyl aminométhane) MERCK réf. : 8382
- Elastase pancréatique SIGMA réf.: E 7885
- Elastine orcéïne SIGMA réf. : E 1500
- Plaque de culture cellulaire 24 puis - FALCON

Les préparations suivantes sont réalisées :

- tampon TRIS - HCl 0,02 M (pH = 8) : la poudre TRIS est dissoute à 0,2 M avec dc l'eau ultra pure Millipore. Le pH est ajusté à 8 avec HCl pur. Puis le produit est dilué au 1/10.
- Solution d'Agar: 2g sont utilisés pour 98 ml de tampon TRIS - HCl 0,02 M (pH=8).

Elastine orcéïne: 150 mg d'élastine sont broyés au mortier dans 1 ml de tampon TRIS - HCl 0,02 M (pH8) puis repris dans 10 ml en final. On centrifugé 10 minutes à 5000 tour/minute et on reprend le culot par 1 ml de tampon TRIS que l'on ajoute à 49 ml de solution d'Agar à 60°C. La solution d'élastine est ensuite maintenue à 60°C.

Produits à tester :

- greffage GPFA 016A (greffage renfermant 12,2% de céramides H03 et 15,2% de chondroïtine sulfate sodique) pré-dissout à 10% dans du propylène glycol, puis dilué au 1/10 dans le tampon TRIS. La solution testée est donc à 1% de greffage.
- céramide H03 testé à la concentration du greffage, c'est-à-dire 12,2%, prédissout à 1,22% dans du propylène glycol, puis dilué au 1/10 dans le tampon TRIS. La solution testée est donc à 0,122% de céramide
- chondroïtine sulfate sodique, testée à la concentration du greffage, c'est-à-dire 15,2% dissout directement dans le tampon TRIS. La solution testée est a 0,152% de chondroïtine sulfate sodique.

Les trois solutions ainsi obtenues, ainsi que le témoin tampon et le témoin propylène glycol, sont introduits dans la solution d'élastine agarose à raison de 1 ml pour 9 ml de gel. 1 ml de cette "solution gel" à tester est introduit par puit et 4 puits par lot de produit à tester sont réalisés.

Introduction de l'élastase :

Un trou est réalisé au centre du gel, précédemment décrit, à l'aide d'un punch biopsie de 4 mm de diamètre et 40 1ll d'une solution d'élastase y sont déposés.

Lecture :

La plaque est ensuite mise à l'incubateur à 37°C avec 5% de $CO_2$ en atmosphère humide. La lecture des diamètres de lyse est effectuée à t0 + 4 jours.

Expression des résultats :

Elle est donnée par la surface de lyse nd1d2/4

Le pourcentage d'inhibition est ainsi calculé :

$$\frac{\text{surface de lyse du lot témoin - surface de lyse du lot traité}}{\text{surface de lyse du lot témoin - surface de lyse du témoin absolu}} \times 100$$

le témoin absolu ne reçoit pas d'élastase, sa surface de lyse est estimée à 0,13 cm2

Les résultats sont consignés dans le tableau ci-dessous :

| VALEURS INDIVIDUELLES (lecture à t0 + 4 jours) | | | | |
|---|---|---|---|---|
| REF | Concentration | Diamètre 1 en $mm^2$ | Diamètre 2 en $mm^2$ | Surface en $cm^2$ |
| GAG | 0.15 | 9 | 9 | 0,64 |
| GAG | 0.15 | 8 | 8 | 0.50 |
| GAG | 0.15 | 8 | 7 | 0.44 |
| GAG | 0.15 | 8 | 7 | 0.44 |
| GPFA016A | 1.0000 | 4 | 4 | 0.13 |
| GPFA016A | 1.0000 | 4 | 4 | 0.13 |
| GPFA016A | 1.0000 | 4 | 4 | 0.13 |
| GPFA016A | 1.0000 | 4 | 4 | 0.13 |
| HO3 | 0.12 | 8 | 8 | 0.50 |
| HO3 | 0.12 | 8 | 7 | 0.44 |
| HO3 | 0.12 | 8 | 7 | 0.44 |
| HO3 | 0.12 | 8 | 7 | 0.44 |
| P. GLYCOL | 10.0000 | 8 | 8 | 0.50 |
| P. GLYCOL | 10.0000 | 9 | 8 | 0.57 |
| P. GLYCOL | 10.0000 | 8 | 8 | 0.50 |
| P. GLYCOL | 10.0000 | 8 | 7 | 0.44 |
| TEMOIN | 0.0000 | 9 | 8 | 0.57 |
| TEMOIN | 0.0000 | 8 | 8 | 0.50 |
| TEMOIN | 0.0000 | 8 | 7 | 0,44 |
| TEMOIN | 0.0000 | 8 | 9 | 0,57 |

| LOTS | Surface de lyse (moyenne en $cm^2$) +/- STD | STD | % D'inhibition |
|---|---|---|---|
| Témoin tampon | 0.52 | 0.031 | - |
| TRIS HCL | 0.5 | 0.047 | - 5.1 |
| GAG 0.15% | 0.455 | 0.015 | - 16.7 |
| GPFA 016A 1% | 0.13 | 0 | -100 |
| Propylène Glycol 10% | 0.502 | 0.026 | -4.6 |

**Légende:**

GAG : chondroïtine sulfate sodique
HO3: céramides HO3
GRFAU16A : greffage

Le greffage apporte donc une remarquable potentialisation de l'activité anti-élastase, comparée aux produits non greffés comme le montre le graphe en annexe (figure 1).

## VII - FORMULATION

Nous exposons ci-après quelques exemples de formules cosmétiques de façon non exhaustive contenant l'association Ceramide-Chondroïtine (mélanges ou greffés) à 0,001 % jusqu'à 5 %.

| BAUME LEVRES | |
|---|---|
| ASSOCIATION CERAMIDE CHONDROITINE | 0,001 à 5 % |
| CASTOR OIL USP | 12,3805 g |

(suite)

| BAUME LEVRES | |
|---|---|
| ISOSTEARYLNEOPENTANOATE | 11,000 g |
| MINERAL OIL, LIGHT | 20,000 g |
| HYDROGENATED CASTOR OIL | 7,000 g |
| OZOKERITE | 10,000 g |
| HYDROGENATED LANOLIN | 10,000 g |
| SPERMACETI WAX | 4,000 g |
| WHITEBEESWAX | 4,000 g |
| OCTYLMETHOXYCINNAMATE | 0,1 à 10,5% g |
| PVP/ HEXADECENE COPOLYMER | 1 à 5% |
| PUR CELLIN OiL | 2,000 g |
| VITAMINEEACETATE | 0,400 g |
| PROPYLPARABEN | 0,100 g |
| PPG-10 LANOLIN ALCOHOL ETHER | 4,000 g |
| (POLYGLYCERYL-4S ISOSTEARATE) CETYL | 0,1 à 2% |
| DIMETHICONECOPOLYOL, HEXYLLAURATE | |
| BHT, CORN OIL | 0,106 g |
| TITANIUMDIOXIDE, OCTYLPALMITATE | 0,1 à 30% |
| MICA | 0,1 à 5% |
| CHERRY FLAVOR 142133 | 0,0135 g |

| SPRAY SOLAIRE | |
|---|---|
| ASSOCIATION CERAMIDE-CHONDROITINE | 0,001 à 5 % |
| HUILE DE TAMANOU | 0,1 à 10 % |
| CINNAMATE | 0,1 à 10 % |
| TI02 | 0,1 à 30 % |
| DIBENZOYLMETHANE | 0,1 à 4 % |
| PEG-5-GLYCERYL STEARATE | 1 à 5 % |
| STEARETH 10 | 0,1 à 5 % |
| P.P.G. 15 STEARYL | 0,1 à 6 % |
| MINERAL OIL | 0,1 à 10 % |
| PARFUM | |
| EAU QSP | 100,000 g |

| COLD CREAM | |
|---|---|
| ASSOCIATION CERAMIDE- CHONDROITINE | 0,001 à à 5 % |
| PARAFFINE LIQUIDE | 20,000 g |
| CETYL/STEARYL2-ETHYLHEXANOATE | 2,000 g |
| PALMITATE DISOPROPYLE | 10,000 g |
| MEIANGE D'ESTERS DE POLYGLYCEROL | 10,000 g |
| CIRES | |
| GLYCERINE CODEX | 4,000 g |
| CONSERVATEUR GD 500 | 2,500 g |
| PARFUM | 0,100 g |
| EAU QSP | 100,000 g |

| EAU SOLAIRE IP 2-4 | |
|---|---|
| ASSOCIATION CERAMIDE-CHONDROITINE | 0,001 à 5% |
| HUILE DE TAMANOU | 0,1 à 10% |
| **EAU SOLAIRE IP 2-4** | |
| EXTRAIT D'HIBISCUS | 1,000 g |
| ALLANTOINE | 0,200 g |
| ACIDE PYROGLUTAMIQUE | 0,300 g |
| PARA METHOXY CINNAMATE D'ETHYL HEXYL | 0,1 à 10,5% |
| DISPERSION HUILEUSE D'OXYDE DE TITANE | 0,1 à 30% |
| BUTYLMETHOXY DIBENZOYLMETHANE | 0,1 à 4% |
| HUILE DE RICIN HYDROGENEE ETHOXYLEE | 3,000 g |
| DMDM | 0,100 g |
| HYDANTOIN/BUTYL-IODO-PROPYNYLCARBAMATE | |
| PARFUM | 0,200 g |
| TRIETHANOLAMINE | 0,400 g |
| EAU PURIFIEE QSP | 100 g |

| SOIN DU VISAGE APRES SOLEIL | |
|---|---|
| ASSOCIATION CERAMIDE-CHONDROITINE | 0,001 à 5% |
| POLYSILOXANE MODIFIEE POLYETHER | 1 à 5% |
| DECAMETHYLCYCLOPENTASILOXANE | 5 à 15% |
| DIMETHYLPOLYSILOXANE 5 CST | 0,1 à 5% |
| PERHYDROSQUALENE | 2,000 g |
| MALEATEDEDIOCTYLE | 2,000 g |
| MELANGE DE CONSERVATEURS | 0,500 g |
| DMDM | |
| HYDANTOIN/BUTYL-ODO-PROPYNYLCARBAMATE | 0,100 g |
| GLYCERINE CODEX | 1 à 25% |
| CHLORURE | |
| DEDIMETHYLDIALIKYLAMMONIUM | 1,300 g |
| MONTMORILLONITE | |
| CHLORURE DE SODIUM OFFICINAL | 1,000 g |
| COLORANT ROUGE CI 16035 | 0,0004 g |
| COLORANT JAUNE CI 19140 | 0,001 g |
| HUILE DE CALLOPHYLUM | 0,100 à 10% |
| ALLANTOINE | 0,100 g |
| PARFUM | 0,100 g |
| EAU PURIFIEE QSP | 100 g |

| PREPARATEUR DE TEINT | |
|---|---|
| ASSOCIATION CERAMIDE - CHONDROITINE | 0,001 à 5% |
| MELANGE D'ORGANOSILICONES | 5 à 20% |
| FLUIDE DC 344 | 5 à 20% |
| MYRISTATEDETRIETHOXY MYRISTYLE | 0,1 à 3% |
| BUTYLHYDROXY TOLUENE | 0,050 g |
| PARAHYDROXYBENZOATEDEPROPYLE | 0,200 g |
| MONOSTEARATEDEGLYCEROL | 0,5 à 3% |
| MELANGE PIGMENT | 1,200 g |
| MELANGE PIGMENTAIRE | 9,000 g |

(suite)

| PREPARATEUR DE TEINT | |
|---|---|
| SULFATE DE MAGNESIUM PH X | 0,500 g |
| GLYCERINE CODEX | 3,000 g |
| DIAZOLIDINYLUREE | 0,200 g |
| EAU PURIFIEE QSP | 55,350 g |

| HUILE SOLAIRE | |
|---|---|
| ASSOCIATION CERAMIDE / CHONDROITINE | 0,001 à 5% |
| HUILEDETAMANOU | 0,1 à 10% |
| EXTRAIT D'HIBISCUS | 0,100 g |
| MONOI | 10 à 90% |
| PARA METHOXY CINNAMATE D'ETHYL HEXYL | 0,100 à 10,5 % |
| BUTYL METHOXY DIBENZOYLMETHANE | 0,100 à 4 %9 |
| DISPERSION HUILEUSE D'OXYDE DE TITANE | 0,100 à 30 % |
| ADIPATE DISOPROPYLE | 8,000 g |
| TRIGLYCERIDE CAPRIQUE CAPRYLIQUE | 2,000 g |
| GALLATE DE PROPYLE | 0,10 g |
| PARFUM | 0,400 g |
| PHENOXYETHANOL | 0,100 g |
| PARAHYDROXYBENZOATEDEMETHYLE | 0,200 g |
| PARAFFINE LIQUIDE LEGERE QSP | 100 g |

| LAIT HYDRATANT APRES SOLEIL | |
|---|---|
| ASSOCIATION CERAMIDE- CHONDROITINE | 0,001 à 0,5 % |
| ALLANTOINE | 0,200 g |
| EXTRAIT DE CALENDUIA | 1,000 g |
| GLYCERINE CODEX | 1 à 10% |
| PARAFFINE LIQUIDE | 1 à 10% |
| LAURYL METHICONE COPOLYOL | 0,5 à 5% |
| DECAMETHYL CYCLOPENTASILOXANE | 0,1 à 20% |
| DIOCTYLSUCCINATE | 0,1 à 5% |
| HUILE DE CARTHAME | 2,000 g |
| CHLORURE DE SODIUM | 2,000 g |
| PARFUM | 0,150 g |
| SILICE | 0,100 g |
| PHENOXYETHANOL | 0,350 g |
| PROPYL PARABEN | 0,200 g |
| HUILE DE CALLOPHYLUM | 0,1 à 10% |
| COLORANT ROUGE CI 16035 | 0,0008 g |
| EAU PURIFIEE QSP | 100 g |

| PREPARATEUR DE BRONZAGE | |
|---|---|
| ASSOCIATION CERAMIDE- CHONDROITINE | 0,001 à 5% |
| HUILE DE CALLOPHYLLUM | 0,1 à 10% |
| COMPLEXE DIHYDROXYACETONE ~ DERIVE DE TYROSINE | 1 à 6 % |

(suite)

| PREPARATEUR DE BRONZAGE | |
|---|---|
| PEG 5 GLYCERYLSTEARATE | 1 à 2% |
| ALCOOL STEARYLIQUE 10 OE | 1 à 5% |
| PEG S STEARYLSTEARATE | 2,500 g |
| PARAFFINE LIQUIDE | 5,000 g |
| TRIGLYCERIDE CAPRIQUE CAPRYLIQUE | 5,000 g |
| DECAMETHYL CYCLOPENTASILOXANE | 1,000 g |
| PARAHYDROXYBENZOATEDEMETHYLE | 0,200 g |
| PARFUM | 0,350 g |
| EAU PURIFIEE QSP | 100,000 g |

| OSMO ACTIF APRES SOLEIL | |
|---|---|
| ASSOCIATION CERAMIDE-CHONDROITINE | 0,001 à 5 % |
| HUILEDETAMANOU | 0,1 à 5% |
| ACIDE DESOXYRIBONUCLEIQUE HPM | 0,100 g |
| HYALURONATE DE SODIUM | 0,001 g |
| D.L. ALPHA BISABOLOL | 0,100 g |
| POLYMERE CARBOXYVINYLIQUE | 0,1 à 1% |
| POLYACRYLATE DE GLYCERINE | 1 à 20% |
| SORBITOL | 1 à 20% |
| DIMETHYLPOLYSILOXANECOPOLYOL | 1,000 g |
| HUILE DE PALME ETHOXYLEE | 0,500 g |
| TRIETHANOLAMINE | 0,800 g |
| PHENOXYETHANOL | 0,400 g |
| HUILE DE RICIN HYDROGENEE ETHOXYLEE | 0,800 g |
| PARFUM | 0,200 g |
| ALCOOL A 95 | 10,000 g |
| PHTALATE D'ETHYLE | 0,061 g |
| GRANULES FR 637 | 0,250 g |
| GRANULES FR 648 | 0,250 g |
| EAU PURIFIEE QSP | 100 g |

| PRODUIT SOLAIRE | |
|---|---|
| ASSOCIATION CERAMIDE- CHONDROITINE | 0,001 à 5 % |
| DIOXYDE DETITANE | 0,1 à 25% |
| PARA METHOXY CINNAMATE D'ETHYL HEXYL | 0,1 à 10,5 % |
| BUTYLMETHOXY DIBENZOYLMETHANE | 0,1 à 4 % |
| HUILE DE CALLOPHYLLUM | 0,100 g |
| C12-15 ALKYLBENZOATE | 2,000 g |
| VOLATILE SILICONE AND POLYDIMETHYLSILOXANE | 2,000 g |
| ALCOOL CETYLIQUE | 0,1 à 2% |
| TRIOLEATE SORBITAN | 0,1 à 5% |
| OLEATEDEDECYLE | 0,1 à 10% |
| PARAHYDROXYBENZOATEDEMETHYLE | 0,300 g |
| PARAHYDROXYBENZOATEDEPROPYLE | 0,400 g |
| DIBROMODICYANOBUTANE | 0,120 g |

(suite)

| PRODUIT SOLAIRE | |
| --- | --- |
| PHENOXYETHANOL | |
| COPOLYMEREACRYLIQUE | 0,01 à 1% |
| POLYMERE CARBOXYVINYLIQUE | 0,1 à 0,5% |
| HYDROXYPROPYLMETHYLCELLULOSE | 0,100 g |
| E.D.T.A. DISODIQUE | 0,100 g |
| PARFUM | 0,400 g |
| TRIETHANOLAMINE | 0,650 g |
| EAU PURIFIEE | 100,000 g |

| GELEE SOLAIRE | |
| --- | --- |
| ASSOCIATION CERAMIDE - CHONDROITINE | 0,001 à 5 % |
| CETEARETH-12 | 1 à 5% |
| CETEARETH-20 | 5 à 10% |
| GLYCERYLSTEARATE | 3 à 10% |
| DECYLOLEATE | 5 à 15% |
| DICAPRYLETHER | 5 à 30% |
| HUILE DE VASELINE EPAISSE | 1 à 20% |
| CINNAMATE | 0,1 à 10% |
| OXYDE DETITANE | 0,1 à 20% |
| DIBENZOYLMETHANE | 0,1 à 4% |
| HUILE DE TAMANOU | 0,1 à 5% |
| CONSERVATEURS | 0,1 à 1% |
| PARFUM | 0,1 g |
| EAU PURIFIEE QSP | 100,000 g |

**Références Bibliographiques**

1/ "Régulation des protéases par les glucocorticoïdes vectorisés par les céramides végétales", Biochemical J. (en cours)

2/ F. FREDINI (1988) : "Inhibition of leucocyte elastase by heparin and its derivatives" BIOCHEM. J. 252 : 515 - 519.

3/ A.K. LEE (1984) Inhibitors, enzymes alld growth factors from shark cartilage.

**Revendications**

**1.** Conjugué de céramides et de glycosaminoglycanes (GAG), caractérisé en ce qu'il est susceptible d'être obtenu par la mise en oeuvre du procédé suivant :

- on fait réagir à température ambiante en phase aqueuse les céramides et les glycosaminoglycanes avec un agent pontant, ledit agent pontant étant choisi parmi les polychlorures d'acides ou les poly-isocyanates, et
- le cas échéant on élimine l'eau, préférentiellement par lyophilisation.

**2.** Conjugué selon la revendication 1, caractérisé en ce que l'agent pontant est le dichlorure de l'acide sébacique.

**3.** Conjugué selon la revendication 1, caractérisé en ce qu'il répond à la formule générale :

$$R5(U-CO-R6-CO-O-R2)n$$

dans laquelle

n est supérieur ou égal à 1
R2-O représente un résidu céramide,
R5 représente un résidu GAG, et
R6 représente un agent pontant, et

lorsque les fonctions alcools primaires du GAG sont impliquées dans la réaction, alors (-U-) représente (-CH2-O-), lorsque les fonctions alcools secondaires du GAG sont impliquées dans la réaction, alors (-U-) représente (-O-), ou lorsque les fonctions amines du GAG sont impliquées dans la réaction, alors (-U-) représente (-NH-).

**4.** Conjugué selon la revendication 3, caractérisé en ce que R6 représente un reste hydrocarboné en $C_1$-$C_{20}$.

**5.** Conjugué selon la revendication 1, caractérisé en ce qu'il est obtenu par la réaction de couplage d'une céramide et d'une glycosaminoglycane (GAG) en présence d'un agent de couplage de type diacide carboxylique, de formule HOOC-R6-COOH dans laquelle R6 représente un reste hydrocarboné en $C_1$-$C_{20}$, le conjugué obtenu comprenant des liaisons céramide/GAG de type covalentes (esters ou amides) et/ou de type ioniques (entre les fonctions amino du GAG et les fonctions acide du céramide) et/ou de type hydrogène, et/ou de type hydrophobe.

**6.** Composition cosmétique caractérisée en ce qu'elle comprend le conjugué selon l'une des revendications 1 à 5 et un support cosmétiquement acceptables.

**7.** Composition selon la revendication 6, caractérisée en ce qu'elle comprend entre 0,001 et 5% de conjugué.

## Patentansprüche

**1.** Konjugat von Ceramiden und Glykosaminoglykanen (GAG), dadurch charakterisiert, daß es erhalten werden kann durch Durchführung des folgenden Verfahrens:

- man läßt bei Raumtemperatur in wäßriger Phase die Ceramide und die Glykosaminoglykane mit einem Verknüpfungsmittel reagieren, wobei das Verknüpfungsmittel ausgewählt wird aus Säurepolychloriden oder Polyisocyanaten, und
- man entfernt gegebenenfalls das Wasser, bevorzugt durch Lyophilisierung.

**2.** Konjugat nach Anspruch 1, dadurch charakterisiert, daß das Verknüpfungsmittel das Dichlorid von Sebacinsäure ist.

**3.** Konjugat nach Anspruch 1, dadurch charakterisiert, daß es der allgemeinen Formel entspricht:

R5(U-CO-R6-CO-O-R2)n

wobei

n größer oder gleich 1 ist
R2-O einen Ceramidrest darstellt,
R5 einen Rest GAG darstellt, und
R6 ein Verknüpfungsmittel darstellt, und wobei,

wenn die primären Alkoholfunktionen von GAG an der Reaktion beteiligt sind, (-U-) ($-CH_2$-O-) darstellt, wenn die sekundären Alkoholfunktionen von GAG an der Reaktion beteiligt sind, (-U-) (-O-) darstellt, oder wenn die Aminfunktionen von GAG an der Reaktion beteiligt sind, (-U-) (-NH-) darstellt.

**4.** Konjugat nach Anspruch 3, dadurch charakterisiert, daß R6 einen $C_1$-$C_{20}$-Kohlenwasserstoffrest darstellt.

**5.** Konjugat nach Anspruch 1, dadurch charakterisiert, daß es erhalten wird durch Kupplungsreaktion eines Ceramids

und eines Glykosaminoglykans (GAG) in Gegenwart eines Kupplungsmittels vom Typ Dicarbonsäure der Formel HOOC-R6-COOH, wobei R6 einen $C_1$-$C_{20}$-Kohlenwasserstoffrest darstellt, wobei das erhaltene Konjugat kovalente Ceramid/GAG-Bindungen (Ester oder Amide) und/oder ionische Bindungen (zwischen den Aminofunktionen von GAG und den sauren Funktionen von Ceramid) und/oder Wasserstoffbindungen und/oder hydrophobe Bindungen umfaßt.

**6.** Kosmetische Zusammensetzung, dadurch charakterisiert, daß sie das Konjugat nach einem der Ansprüche 1 bis 5 und einen kosmetisch verträglichen Träger umfaßt.

**7.** Zusammensetzung nach Anspruch 6, dadurch charakterisiert, daß sie zwischen 0,001 und 5 % des Konjugats umfaßt.

**Claims**

**1.** Conjugate of ceramides and of glycosaminoglycans (GAG), characterized in that it can be obtained by carrying out the following process:

- the ceramides and the glycosaminoglycans are reacted, at room temperature and in an aqueous phase, with a bridging agent, the said bridging agent being chosen from polyacid chlorides and polyisocyanates, and
- where appropriate, the water is removed, preferably by freeze-drying.

**2.** Conjugate according to Claim 1, characterized in that the bridging agent is sebacic acid dichloride.

**3.** Conjugate according to Claim 1, characterized in that it corresponds to the general formula:

R5(U-CO-R6-CO-O-R2)n

in which

n is greater than or equal to 1,
R2-O represents a ceramide residue,
R5 represents a GAG residue, and
R6 represents a bridging agent, and

when the primary alcohol functions of the GAG are involved in the reaction, then (-U-) represents (-$CH_2$-O-), when the secondary alcohol functions of the GAG are involved in the reaction, then (-U-) represents (-O-), or when the amine functions of the GAG are involved in the reaction, then (-U-) represents (-NH-).

**4.** Conjugate according to Claim 3, characterized in that R6 represents a $C_1$-$C_{20}$ hydrocarbon residue.

**5.** Conjugate according to Claim 1, characterized in that it is obtained by the coupling reaction of a ceramide and of a glycosaminoglycan (GAG) in the presence of a coupling agent of dicarboxylic acid type, of formula HOOC-R6-COOH in which R6 represents a $C_1$-$C_{20}$ hydrocarbon residue, the conjugate obtained comprising ceramide/GAG bonds of covalent type (esters or amides) and/or of ionic type (between the amino functions of the GAG and the acid functions of the ceramide) and/or of hydrogen type, and/or of hydrophobic type.

**6.** Cosmetic composition, characterized in that it comprises the conjugate according to one of Claims 1 to 5 and a cosmetically acceptable support.

**7.** Composition according to Claim 6, characterized in that it comprises between 0.001 and 5% of conjugate.

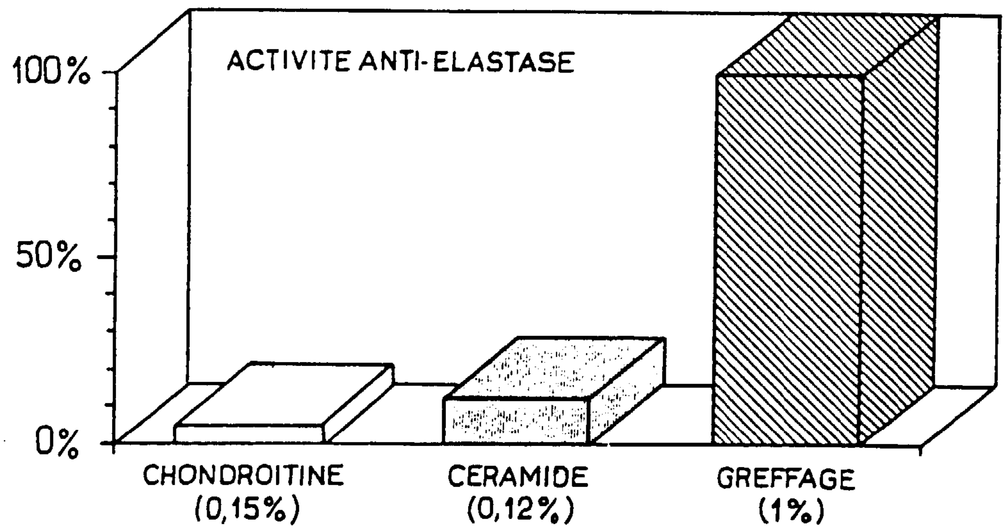
100%
50%
0%
ACTIVITE ANTI-ELASTASE
CHONDROITINE (0,15%)
CERAMIDE (0,12%)
GREFFAGE (1%)

FIG.1